# EUROPEAN PATENT SPECIFICATION

(11) **EP 0 794 787 B1**
(45) Date of publication and mention of the grant of the patent: **05.02.2003**
(21) Application number: 95940768.5
(22) Date of filing: 30.11.1995
(51) Int. Cl.: A61K 31/70, A61P 31/00

(54) **COMPOSITIONS FOR THE TREATMENT OF PARASITIC AND FUNGAL INFECTIONS**
ARZNEIMITTEL ZUR BEHANDLUNG VON PARASITISCHEN UND PILZARTIGEN INFEKTIONEN
COMPOSITIONS DESTINEES AU TRAITEMENT DES PARASITOSES ET DES INFECTIONS FONGIQUES

(30) Priority: 30.11.1994 US 351067; 30.11.1994 US 351068
(43) Date of publication of application: 17.09.1997
(73) Proprietor: THE UNIVERSITY HOSPITAL, Boston, MA 02118 (US)
(72) Inventor: MCCAFFREY, Ronald, P., Needham, MA 02198 (US); WIGZELL, Hans, L., R., S-126 54 Hagersten (SE); SUGAR, Alan, M., Wellesly, MA 02181 (US)
(74) Representative: Tombling, Adrian George
(86) International application number: US9515116
(87) International publication number: WO96016664

(56) References cited:
- CHEMICAL ABSTRACTS, vol. 85, no. 15, 11 October 1976 Columbus, Ohio, US; abstract no. 103786, & BIOCHEM. PHARMACOL., vol. 25, no. 12, 1976 pages 1441-1444, D.G.JOHNS ET AL. 'ENHANCEMENT OF THE BIOLOGICAL ACTIVITY OF CORDYCEPIN (3'-DEOXYADENOSINE) BY THE ADENOSINE DEAMINASE INHIBITOR 2'-DEOXYCOFORMYCIN.'
- CHEMICAL ABSTRACTS, vol. 86, no. 19, 9 May 1977 Columbus, Ohio, US; abstract no. 133421, & PHARMACOLOGY, vol. 15, no. 1, 1977 pages 84-89, R.H.ADAMSON ET AL. 'ENHANCEMENT OF THE BIOLOGICAL ACTIVITY OF ADENOSINE ANALOGS BY THE ADENOSINE DEAMINASE INHIBITOR 2'-DEOXYCOFORMYCIN.'
- DATABASE WPI Week 7750 Derwent Publications Ltd., London, GB; AN 77-89082Y [50] & JP,A,52 130 991 (YAMASA SHOYU KK) 2 November 1977

## Description

### Rights in the Invention

This invention was made with United States Government support under grant number CA 52020, awarded by the National Cancer Institute of the National Institutes of Health, and the United States Government has certain rights in the invention.

### Background of the Invention

### 1. Field of the Invention

The invention relates to compositions for the prevention and treatment of infections caused by fungal and fungal-like organisms. Useful compositions contain an adenosine or adenosine derivative and, optionally, a deaminase inhibitor. Compositions may be used *in vivo* to treat patients and also *in vitro* to inactivate or destroy infectious organisms in cultured cells, in fluids such as blood and blood products, in biological products derived from cells and in foods. This invention also relates to compositions and methods for treating or preventing neoplastic disorders.

### 2. Description of the Background

Mycology is the study of fungi, commonly considered the yeasts and molds. Medical mycology concerns itself with those organisms which cause diseases in humans and other animals. Most of the clinically important mycotic organisms are eukaryotic, however, there is a unique group of bacteria that grow as branched, long or short filaments just like the fungi. These fungal-like prokaryotes are the actinomycetes and they comprise at least eight different families.

Most of the actinomycetes are harmless although a few are pathogenic to humans. The most notable of those are *Nocardia asteroides* and *N. brasiliensis*, the causative agents of nocardiosis, and *Actinomyces israelii*, the causative agent of actinomycosis. Nocardiosis occurs most frequently in the respiratory tract and manifests itself as a respiratory disorder. A typical disease course involves the development of one or more lung abscesses which enlarge over time forming cavities. Organisms spread from the lungs into the blood and establish themselves throughout the body forming abscesses. These abscesses destroy soft tissues and bone and can erupt through the skin. Once metastasis has occurred there is little chance for recovery. Treatments typically involve systemic administration of sulfadiazine, but the prognosis is always poor. Patients with nocardiosis are usually immunocompromised and will respond poorly to any treatment. Resistance to treatments also tends to be fairly common.

Another of the fungal-like bacteria is *Actinomyces israelli*. These organisms, unlike *Nocardia* exist as a part of the normal flora of most mammals including humans. Infections tend to initiate from an endogenous source. Cervicofacial actinomycosis occurs as a result of trauma to the mucous membranes of the head. Early symptoms include pain with an associated lumpy-swelling of mucous membranes of the jaw. As the swelling subsides, organisms spread throughout the body. Sites where infections take hold can extend through the skin forming abscesses. These organisms often infect the oral cavity in patients with poor dental hygiene.

Abdominal actinomycosis is typically caused by ingestion of organisms into a weak or debilitated gastrointestinal tract. Infections can perforate the intestine forming ulcerations which extend into the major organs such as the liver, urogenital system and bone. Penicillin and its derivatives are the treatment of choice, but chlortetracycline, chloramphenicol, streptomycin, sulfadiazine and other sulfonamides have been shown to be useful.

True fungi are eukaryotic organisms which grow as single cells or long-branched, filamentous structures. Most have both a sexual and an asexual stage. There are few major classes which cause diseases in humans although, to a large degree, their complete pathology is best described as unclear. Many produce enzymes and toxins which can be allergenic to a host, while others cause physical damage and destruction to tissues.

Fungal pathogens of humans can be divided into two categories. Those that infect superficially and those that invade and infect the deep organs and tissues. Four important families which are pathogenic in humans are the *Zygomycetes,* the *Basidiomycetes*, the *Ascomycetes* and the *Deuteromycetes*. The *Zygomycetes* all lack regular septa (cross-walls) in their hyphal filaments and cells typically possess multiple nucleic. *Zygomycetes* are a typical opportunistic and nosocomial infections (mucormycosis) caused by various species of breadmold fungi including the genera *Mucor, Absidia, Rhizopus, Cunninghamella* and other *Mucorales*. A few cause human disease which can become serious in the immunosuppressed and other immunocompromised individuals. As most patients with mucormycosis have a serious underlying disorder, effective treatment is often impossible.

Another large group of pathogenic fungi are *Deuteromycetes* which include a large number of organisms which typically colonize superficial lesions of the skin. Cutaneous infections tend to be restricted to keratinized tissues, but the *Deuteromycetes* can also cause deep-seated systemic infections that can become life-threatening.

Those organisms which typically infect superficially include *Trichophyton rubrum*, *T. mentagrophytes* and *T. floccosum*. These are the organisms most often responsible for tinea pedis, also referred to as athlete's foot. An estimated 30%-70% of the population of the Western world is believed to be at least subclinically infected. Chronically infected patients can develop hypersensitivity and a severe allergic response. Most have to deal with milder symptoms which include itchy peeling skin and ulcerations and cracking of the dermis.

Ringworm, tinea corporis, may occur anywhere on the body and is not restricted to humans. Various species of dermatophytes cause ringworm including *T. mentagrophytes* and *T. rubrum*, both of which can be spread from animals to humans. Most infections of normal healthy individuals will clear with time. Ringworm of the nail, tinea unguium (onychomycosis), is a common infection of toe nails. Although most infections are minor, tinea unguium is always associated with systemic dermatophyte infections.

Tinea capitis is a common skin infection that appears as red scaly lesions on the body with associated hair loss (alopecia) of the infected area. The most common etiological agents are *T. tonsurans, Microsporum audouini* and *M. canis*. Inflammatory reactions can produce deep ulcers with permanent alopecia.

Treatments for these diseases are all fairly similar and involve topical applications of long-chain fatty acids, salicylic acid, selenium sulfide and sulfur in ointments. One current treatment is topical griseofulvin, an antibiotic produced by a penicillium-type mold. Treatments usually require weeks or months and in certain cases even years.

Deep tissue fungal infections are all very serious. In many cases, such a diagnosis is a death sentence. Most human disease associated with fungal and fungal-like infections are attributed to the *Saccharomycetes* and other members of the family *Ascomycetes*. These organisms reproduce as single- or multi-celled conidia, which are formed in conidiophores.

Species of the genera *Candida* are the most frequent causes of human fungal diseases. All appear as non-branching, boxcar-like chains of tubular cells called pseudohyphae that bud, forming blastospores. These organisms colonize the oral cavity, gastrointestinal tract and vagina of many otherwise healthy individuals. These fungi can become pathogenic with a change in, for example, the pH or chemical make-up of their immediate local environment, or by antibiotic suppression of the surrounding organisms. Some of the species commonly associated with disease include *Carrdida krusei, C. glabrata, C. parapsilosis* and *C. tropicalis*. The most common species responsible for disease in humans is *Candida albicans.*

The most common forms of candidiasis are associated with mucosal, oral and vaginal infections. However, severe burns and surface wounds in patients with diabetes mellitus are frequently infected with *C. albicans.* Candidiasis of the oral cavity is referred to as thrush. It is common in bottle-fed newborns and as a complication of gum diseases. Vaginal candidiasis is common in many healthy women taking oral contraceptives or antibiotics, or during pregnancy. Cutaneous eczematoid candidiasis can arise in any area of the skin which remains warm and moist for long periods of time. All cases tend to respond well to drug therapy such as administration of wide-spectrum anti-fungal drugs. Cutaneous infections also respond readily to treatment of the underlying condition. Chronic infections with no obvious associated cause are more difficult to treat and may persist because of an underlying defect in T cell-mediated immunity or the presence of a polyendocrine deficiency in the patient such as hypoparathyroidism, hypoadrenalism or hypothyroidism.

In most cases, severe invasive candidiasis with visceral dissemination is indicative of a serious underlying disorder associated with immunosuppression or neutrophil depletion. Invasive candidiasis of the deep tissues can initiate from deep incisions or direct inoculation into the blood. Sources include intravenous lines, catheters, intravenous drug abuse, and all forms of surgical procedures. Infections are common in the immunocompromised and immunosuppressed who can easily become septic. Although not usually as rapidly progressive as bacterial sepsis, candidal sepsis has similar clinical manifestations including disseminated intravascular coagulation (DIC), shock, renal failure, lung and liver complications, and associated cardiac problems. Renal involvement is seen in over 90% of invasive cases. Prognosis is often poor.

Pathogenic fungal and fungal-like organisms tend to establish themselves, as would be expected, in the sinuses, orbit and brain, giving rise to, for example, rhinocerebral mucormycosis. In the lungs, candida lesions are often extensive and hemorrhagic. Meningitis, intracerebral abscesses, enteritis, arthritis and osteomyelitis are some common presentations of fungal and fungal-like diseases. Nevertheless, in certain cases, infections may cause no inflammatory reaction or a suppurative response.

Although a number of specific stains and culturing conditions have been developed, a definitive diagnosis of candidiasis is often difficult to make and to confirm, as the organisms are nearly ubiquitous over the entire body. Presence anywhere within the body cavity however is always definitive evidence of infection.

*Aspergillus fumigatus, A. niger* and other species of *Aspergillus* rank very closely behind *Candida* as common causes of fungal infections. Certain species can damage an otherwise healthy individual by mere inhalation of spores. Such individuals are hypersensitive to the spores and manifest an extreme inflammatory reaction. Other species produce damaging toxins. *A. flavus*, although not commonly infectious to humans, produces a liver carcinogen frequently found in many edible foods such as vegetables, fruits, breads, dairy products and even meats.

A diagnosis of colonizing aspergillosis implies growth of fungus in the pulmonary tract with minimal tissue invasion. Proliferating masses of fungal hyphae called fungus balls can be seen as brownish masses in pulmonary cavities. Invasive aspergillosis is an opportunistic infection typically found only in immunocompromised and debilitated hosts. Lesions occur primarily in the lungs, but also in the brain, kidneys and heart valves. Pulmonary lesions appear as necrotizing pneumonia with sharply delineated, rounded gray foci. These infections are rapidly progressive and life-threatening.

Another of the deep-tissue fungal infections is cryptococcosis, typically caused by *Cryptococcus neoformans*. These organisms can cause infections in healthy individuals exposed to massive numbers of spores, but most infections are restricted to immunocompromised patients. Patients with Hodgkin's disease and other leukemias and lymphomas may have associated cryptococcus infections. Pulmonary cryptococcus can be distinguished from other lung infections by definitive identification of the fungus in sputum or at biopsy using the latex cryptococcal antigen test (LCAT).

Blastomycosis, typically an infection of *Blastomycetes dermatitis*, is a chronic infection that can be characterized by focal suppurative and granulomatous lesions most commonly in the lungs and skin. Any area of the body can be affected including the internal organs. Symptoms can resemble tuberculosis or other mycoses and even malignant ulcers. Minor infections can resolve in healthy individuals, but late stage diseases require antifungal therapy.

Paracoccidiodomycosis, or South American blastomycosis is caused by *Paracoccidioides brasiliensis.* It is endemic in certain areas from Mexico to Argentina. The disease usually presents itself as extra-pulmonary lesions of the mouth, nose and larynx that spreads to local lymph nodes. All organs and organ systems are susceptible. Again, most short term treatments are unsuccessful. Therapy for years often can control the disease.

Coccidioidomycosis, another fungal disease, is caused by the inhalation of spores of the organism, *Coccidioides immitis*. These organisms cause an acute and chronic infection, similar to tuberculosis and primarily in the lungs. Approximately 60% of infected patients are asymptomatic. Another 10% present symptoms of mild coughing or fever (San Joaquin valley fever). Only about 0.2% of infected patients have more severe symptoms such as chills, fever, weight loss and a general wasting. Central nervous system involvement and bone destruction can occur in the very serious cases. Such individuals are believed to have an unidentified cell-mediated immune deficiency.

Infections caused by- *Histoplasma capsulatum* are endemic and rival the frequency of tuberculosis in the United States. This organism can be acquired by the inhalation of infected dust contaminated with bird or bat droppings. As with most fungal infections, pulmonary involvement is most common which can be asymptomatic or spread to any organ or tissue when unchecked by a healthy immune system.

The treatment of choice for almost all forms of fungal infections is amphotericin B. It can only be given intravenously and in many cases, amphotericin B is the only treatment available. Unfortunately, many organisms are not sensitive to its effects. Oral azoles such as mycoconazole, ketoconazol, fluconazole and itraconazole, which are less toxic than amphotericin B and can be administered orally, are increasingly being used to treat certain of the mycoses.

Although there has been some success with current treatments of both parasitic and fungal infections, the diseases they cause remain serious sources of morbidity and mortality in the world.

### Summary of the Invention

The present invention overcomes the problems and disadvantages associated with current strategies and designs and provides new methods for the treatment and prevention of diseases and disorders related to infection by eukaryotic organisms.

One embodiment of the invention is directed to compositions containing an adenosine derivative and, optionally, a deaminase inhibitor. Examples of useful adenosine derivatives include cordycepins, cordycepin analogs and derivatives, and modifications of these molecular structures. Examples of useful deaminase inhibitors include coformycins, coformycin analogs and derivatives and modifications thereof. Preferably, the adenosine derivative is in an excess over the deaminase inhibitor. These compositions can be used *in vivo* for the treatment of mammals or *in vitro* for the treatment of biological products.

Another embodiment of the invention is directed to compositions for the treatment of patients suspected of being infected with fungal and fungal-like organisms comprising the administration to the patient of an adenosine derivative and, optionally, a deaminase inhibitor. Treatments may be administered parenterally, sublingually, enterally, by pulmonary absorption or by topical application. Adenosine may be administered simultaneously with or shortly before or after the deaminase inhibitor. Patients which may be successfully treated include most mammals such as humans. These methods can result in complete elimination of infectious organisms from the patient and resolution of all symptoms associated with infection.

Another embodiment of the invention is directed to methods for the treatment of a biological product such as living cells or tissues suspected of or at risk of being infected with a fungal or fungal-like organism to prevent or eliminate the contamination. Treatments can be applied to, for example, whole blood, fractionated blood, plasma, serum, and transplantable organs, and most typically involves administration of an adenosine derivative and, optionally, a deaminase inhibitor.

Another embodiment of the invention is directed to methods for the treatment of a biological product such as material derived from living cells or tissues suspected of being contaminated with a fungal or fungal-like organism, or foods to prevent or eliminate the contamination. Treatments can be applied to products purified from cells such as cytokines, immune system regulators, recombinant proteins and blood products. Treatments involve administration of an adenosine derivative and, optionally, a deaminase inhibitor.

Other embodiments and advantages of the invention are set forth, in part, in the description which follows and, also in part, will be obvious from this description or may be learned from the practice of the invention.

### Description of the Figure

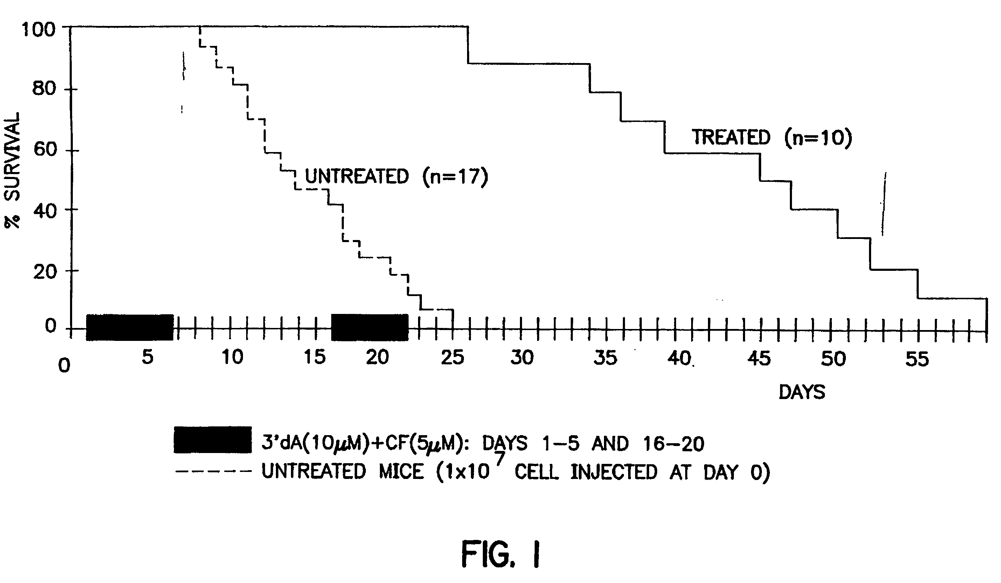

- Figure 1: Event free survival in the TdT-positive SCID mouse leukemia model using NALM-6 cells.

### Description of the Invention

As embodied and broadly described herein, the present invention is directed to compositions and methods for the treatment and prevention of fungal and fungal-like infections and parasitic infections of eukaryotic organisms.

One embodiment of the invention is directed to pharmaceutical compositions. Compositions contain an adenosine derivative comprising a five-membered pentose ring coupled to a purine. Preferably, the adenosine derivative has the formula: 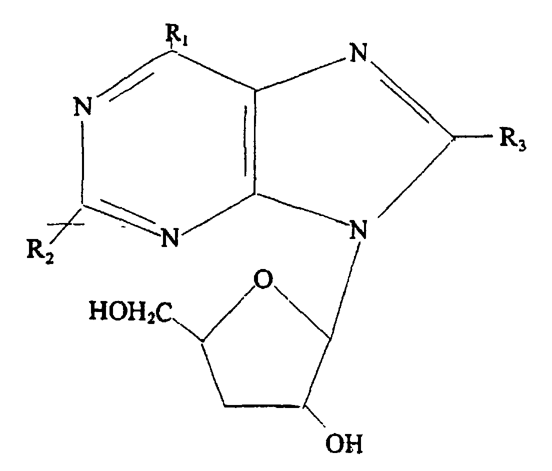 wherein R₁, R₂ and R₃ are each a chemical moiety such as hydrogen, hydroxyl, halide, alkyl or alkoxyl, amine or amide, sulfhydral, nitryl, phosphoryl, sulfinyl or sulfonyl, or a combination or derivative of these chemical moieties. Additionally, R₁, R₂ and R₃ and may be the same or different. More preferably, R₁ is OH, SH, SCH₃ or NHCH₃; R₂ is H, F, Cl, Br, I or NH₂; and R₃ is H, F, Cl, Br, I or CH₃. Examples of useful adenosine derivatives include cordycepin (3'-deoxyadenosine) and cordycepin derivatives. Derivatives may be identified from existing compounds by testing as herein described or as known in the art. Derivatives may also be organically or enzymatically created with techniques such as, for example, rational drug design and empirical screening using the disclosures provided herein and the knowledge of one of ordinary skill in the art.

Compositions of the invention are also effective against a wide variety of fungal and fungal-like organisms. Examples of infections which can be treated by compositions of the invention include the fundal diseases candidiasis, tinea pedis, tinea corporis and tinea capitis, aspergillosis, mucormycosis, phaeohyphomycosis, cryptococcosis, coccidioidomycosis, blastomycosis, histomycosis, paracoccidiodomycosis and the *Dematiaceous* infections, and the fungal-like diseases nocardiosis and actinomycosis.

Treatable infections can occur in blood, tissues, the lymphatic system, the respiratory and gastrointestinal tract, the major organs and organ systems, and the dermis. Fungal organisms which are sensitive to treatments include *Candida krusei, C. glabrata, C. albicans* and *C. tropicalis*, the causative agents of candidiasis, and *Trichophyton rubrum, T. mentagrophytes, T. tonsurans, Microsporum audouini, M. canis* and *T. floccosum*, the causative agents of tinea pedis, tinea corporis and tinea capitis. Compositions can also be used to treat infections by *Nocardia asteroides* and *N. brasiliensis, Actinomyces israelii,* species of the genera *Mucor, Absidia, Rhizopus, Cunninghamella* and unrelated *Mucorales*, *Aspergillus fumigatus, A. flavus, A. niger* and other species of *Aspergillus, Cryptococcus neoformans, Paracoccidioides brasiliensis, Coccidioides immitis, Blastomycetes dermatitis* and *Histoplasma capsulatum.*

The mechanism of action of an adenosine derivative of the invention is directed to the disruption of enzymatic processes of the organism that relate to the metabolism of purine, purine derivatives and purine-like chemical structures. These processes are basic reactions that exist in all organisms. Purines and related compounds are involved in biosynthetic and enzymatic pathways such as nucleotide biosynthesis including the synthesis of ATP and GTP, nucleic acid and protein biosynthesis, intracellular and extracellular cellular signaling, mitosis, meiosis, DNA replication, RNA transcription, folate metabolism, the activities of protein kinases A and C, the purine cycle, excretion, absorption and secretion, and other processes considered fundamental to life. Parasites and fungal organisms are acutely sensitive to interference of these processes, and thus, compositions that specifically target these reactions can be used to selectively eliminate an infection or ameliorate the pathogenic symptoms associated with an infection, for example by reducing parasitemia, without unnecessary injury to the host.

Compositions of the invention contain an adenosine derivative at a concentration of between about 1.0 nM to about 10 mM. Such compositions may be concentrated forms of the active ingredients or may be unconcentrated and suitable for immediate use. Alternatively, compositions which are liquids (vol./vol.) or solids (wt./wt.), may contain active components at between about 0.001 % to about 100%, preferably between about 0.01 % to about 10.0%, and more preferably between about 0.1% to about 5.0%, but may be further diluted, if necessary, such as for prolonged direct contact with skin or other bodily tissues where such contact would be harmful to the patient. Compositions may also contain a pharmaceutically acceptable carrier to facilitate administration or introduction to a patient or biological product, to maintain or increase physiological stability, or to facilitate storage or half-life of the composition. Useful pharmaceutically acceptable carriers include water, various oils, salts, saccharides and polysaccharides, glycerols, collagens and combinations and modifications of these substances. Compositions of the invention may optionally include flavoring agents and other agents that may be necessary or desirable to increase shelf-life, such as preservatives, anti-oxidants and other components advantageous for manufacture and distribution of the composition.

Compositions of the invention may optionally contain a deaminase inhibitor such as the adenosine deaminase inhibitor coformycin and derivatives of coformycin such as deoxycoformycin. Certain adenosine derivatives are enzymatically deaminated into inactive inosine by deaminases which may be present in the patient or the article being treated. The addition of such an inhibitor prevents deamination of the adenosine derivative, and thus, its inactivation. Further, deaminase inhibitors have an effective role in treatment. For example, deaminase inhibitors diminish the metabolism of deoxyadenosine to deoxyadenosine triphosphate and alter the activity of certain kinases and polymerases. These activities may also have a useful effect against the parasite. Deaminase inhibitors such as coformycin and deoxycoformycin are safe and non-cytotoxic at useful concentrations. Compositions of the invention which include a deaminase inhibitor contain this component at a concentration of between about 0.1 nM to about 1.0 mM. Alternatively, compositions which are liquids (vol./vol.) or solids (wt./wt.) may contain between about 0.001% to about 100%, preferably between about 0.01% to about 10.0%, and more preferably between about 0.1% to about 5.0% of the deaminase inhibitor, but may, if necessary, be further diluted.

Compositions of the invention containing both an adenosine derivative and a deaminase inhibitor will preferably have an excess of the adenosine derivative over the inhibitor. The adenosine derivative will preferably be in a two- to twenty-fold excess over the deaminase inhibitor and more preferably in a five- to ten-fold excess. The deaminase inhibitor may be administered prior to, subsequent to, or simultaneous with the adenosine derivative. The composition may contain these two ingredients in a mixture to be administered together or the two may be kept separate and administered separately. As both adenosine derivatives and deaminase inhibitors can be effectively dissolved in aqueous solutions, they can be easily utilized as mixtures in liquid or solid form.

Another embodiment of the invention is directed to compositions wherein the adenosine derivative is coupled to the deaminase inhibitor. Coupling may be by ionic bonding such as hydrogen bonding, covalent bonding and affinity bonding. Preferably, the bond formed is stable when used *in vivo* or *in vitro*. In compositions where the adenosine derivative and the deaminase inhibitor are separate, it may be difficult to effectively administer both components. Compositions wherein the two components are coupled together are effective for treating or preventing an infection because the deaminase inhibitor is effective in all locations where the adenosine derivative is active. Compositions can be used *in vivo* to treat human patients infected or suspected of being infected, or *in vitro* to treat a biological product suspected of being contaminated.

The patient being treated is administered a therapeutically effective amount of a composition of the invention. Patients which can be treated include mammals such as a dog, cat, horse, cow, cattle, pig, sheep, goat, rodents, camels or chicken, or a wild animal, but is preferably a human. Zoo animals such as monkeys (primates) and penguins also tend to acquire parasitic and/or fungal infections which are treatable with compositions of the invention. For example, passage through a non-human host is often a requisite part of the life cycle of a parasite. Elimination of the organism in the animal host is an effective means for eliminating or preventing infections in humans. Further, as most fungal organisms can infect across species and genera boundaries, elimination of the fungal organisms in the animal is sometimes an effective means for eliminating or preventing infections in humans. As compositions of the invention are non-toxic at effective concentrations, compositions can safely be used to treat both human and non-human patients.

Administration may be to an adult, an adolescent, a child, a neonate or an infant, or even to a patient *in utero*. Dosages range from between about 1 ng/kg patient weight to about 10 mg/kg patient weight. Administration of the composition may be for a short term, continuous or sporadic as necessary. Patients with a suspected or diagnosed infections may only require treatments for short periods of time or until the infection has proceeded to remission or has been effectively eliminated. Alternatively, to effectively eliminate certain organisms, administration may require long term treatments such as for months or years. As compositions of the invention are generally safe and non-toxic at required dosages, this does not present a problem.

Compositions are administered in a manner which is most useful for the infection being treated. Useful methods of administration include oral, parenteral, sublingual, rectal or enteral administration, pulmonary absorption or topical application. Parenteral administration may be by intravenous injection, subcutaneous injection, intramuscular injection, intra-arterial injection, intrathecal injection, intraperitoneal injection or direct injection or other administration directly to the site or sites of infection. Injectable forms of administration are sometimes preferred for maximal systemic effect against systemic infections and infections of the respiratory tract and the deep tissues. When long term administration by injection is necessary medi-ports, in-dwelling catheters, or automatic pumping mechanisms may be used. These devices provide direct and immediate access to the arteries in and around the heart, the lungs and other major organs and organ systems. Such devices are useful for treating parasitic diseases that infect organs and organ systems such as the blood and tissue dwelling nematodes, malaria, trypanosomiasis and leishmania.

Another effective method of administering compositions to infectious sites may be by transdermal transfusion such as with a transdermal patch and other means of direct contact with affected tissues, or by administration to an internal infection through an incision or some other natural or artificial opening into the body. Compositions may also be administered to the nasal passages as a spray. Diseases localized to the respiratory tract, the head and brain area are treatable in this fashion as arteries of the nasal area provide a rapid and efficient access to the upper areas of the body. Sprays also provide immediate access to the pulmonary system and the bloodstream. Nasal sprays are a preferable method for administering compositions to these areas. Access to the gastrointestinal tract is also achievable using oral, enema, or injectable forms of administration. Compositions may be administered as a bolus injection or spray, or administered sequentially over time (episodically) such as every two, four, six or eight hours, every day (QD) or every other day (QOD), or over longer periods of time such as weeks to months for as long as it takes the infection to resolve or for the patient's own system to be able to overcome the infection.

Orally active compositions are preferred as oral administration is usually the safest, most convenient and economical mode of drug delivery. Oral administration may often be the most effective administer compositions directed to parasites of the gastrointestinal tract. Oral administration can be disadvantageous because compositions are poorly absorbed through the gastrointestinal lining. Compounds which are poorly absorbed tend to be highly polar. Consequently, compounds which are effective, as described herein, may be made orally bioavailable by reducing or eliminating their polarity without significantly compromising their functional activity. This can often be accomplished by formulating a composition with a complimentary reagent which neutralizes its polarity, or modifying the compound with a neutralizing chemical group. Oral bioavailability is also a problem because drugs are exposed to the extremes of gastric pH and gastric enzymes. These problems can be overcome in a similar manner by modifying the molecular structure to be able to withstand very low pH conditions and resist the enzymes of the gastric mucosa such as by neutralizing an ionic group, by covalently bonding an ionic interaction, or by stabilizing or removing a disulfide bond or other relatively labile bond.

When the composition is administered orally, it may be in the form of a liquid, a spray, a powder, a pill, a tablet or a capsule. To facilitate oral administration, compositions of the invention will preferably include flavoring agents and other agents to increase shelf-life or as may be required or desired for ease of transportation and effective delivery.

Administration by any method can be accurately quantitated by measuring levels of the composition from a sample of bodily fluid such as blood, serum or plasma. Effective serum levels of active components of the invention such as the adenosine derivative or deaminase inhibitor are between about 0.01 nM to about 1.0 mM, preferably between about 1.0 nM to about 0.1 mM, and more preferably between about 10.0 nM to about 50.0 nM. Effective levels of the deaminase inhibitor may be between two- and twenty-times lower than effective levels of the adenosine derivative. When applied by direct contact, effective levels of active ingredient may sometimes be analyzed by determining concentration of the composition in the areas which are in close contact with the area of application. For example, when applied topically to the skin, effective levels may be determined from fluid or tissue samples of the dermal tissues within a few centimeters under the area of application. In such cases, composition strength may be predetermined and used as a concentrated solution.

Compositions can be administered by oral or enema formulations, or by rectal irrigation to maximize their contact with and effectiveness on the gastrointestinal system. In such cases, dosages are between about 1% to about 20% (vol./vol.) or between about 1 mM to about 100 mM. Doses are administered until symptoms improve sufficiently for the patient's immune system to resolve the infection or the parasite is killed or eliminated. Multiple and frequent dosing is not problematic because the compounds of the invention are safe, non-toxic and physiologically stable.

Positive effects of treatment include a reduction of organism load or parasitemia, death or inactivation of the organism, decreased infectivity or spore-forming ability of the organism, or elimination of the organism from the body. Preferably, the patient has an organism load (e.g. parasitemia) which is reduced at least 100-fold, more preferably 1000-fold, and even more preferably is undetectable after treatment. The presence of infection may be determined by growing organisms from biological samples obtained from the patient suspected to be infected into suitable cultures and counting the organisms which can be grown. Alternatively, biological samples are obtained from selected ares of the patient suspected to be infected and the numbers of organisms visualized directly or indirectly under a microscope or other suitable devise and counted. Fluorescent-conjugated antibodies may also be used in, for example, an ELISA or other markers to detect specific mycotic or parasitic antigens, or anti-antigen antibodies in a biological sample to determine the degree of infection and the effect of treatments.

Another embodiment of the invention is directed to compositions of the invention which can be used in combination with other agents to maximize the effect of the compositions in an additive or synergistic manner. Agents which may be effective in combination with the compositions of the invention include other drugs and treatments which are known or suspected to have a positive effect against the organism. Examples of additional agents known to be effective against one or more pathogenic fungal and fungal-like organisms include flucytosine, mycoconazole, fluconazole, itraconazole, ketoconazole and griseofulvin, antibiotics such as amphotericin B, sulfadiazine, penicillin, chlortetracycline, chloramphenicol, streptomycin and other sulfonamides, and derivatives, modifications and combinations of these agents.

Therapies using various combinations of these agents would be safe and effective therapies against infections. Combinations of therapies may also be effective in inducing suppression or elimination of an infection such as compositions of the invention plus radiation therapy, toxin or drug conjugated antibody therapy using monoclonal or polyclonal antibodies directed against, for example, the parasite, the fungus, infected cells, gene therapy or specific anti-sense therapy. Effects may be additive, logarithmic, or synergistic, and methods involving combinations of therapies may be simultaneous protocols, intermittent protocols or protocols which are empirically determined.

Another embodiment of the invention is directed to compositions described above which can be used prophylactically. For example, patients exposed to areas where a parasitic disease or a fungal or fungal-like disease is endemic may be continuously treated with compositions to prevent a parasitic infection from taking hold. Patients who have been genetically screened and determined to be at high risk for the future development of an infection may also be administered compositions, possibly beginning at birth and possibly for life. Administration may be by any means described and dosages may be reduced in comparison to dosages required for treatment. Both prophylactic and therapeutic uses are readily acceptable because these compounds are generally safe and non-toxic at useful dosages.

Another embodiment of the invention is directed to methods for the treatment of biological products suspected of being contaminated with a fungal or fungal-like organism. Products which can be treated or pretreated include whole blood, fractionated blood, plasma, serum, transplantable organs, living cells including bone marrow, stem cells, primary cells surgically obtained and established cell lines, and products derived from living cells. Products which can be derived from living cells include blood products such as insulin, the blood clotting factors (*e.g.* Factor V, VIII, VIII, IX, X, XI, XII), cytokines (*e.g.* interferon α, β or γ; the II-1, II-2, Il-3, etc.), complement proteins, antibodies, immune system regulators, recombinant proteins and other macromolecular products.

Treatment involves contact of the biological product with a solution comprising an adenosine derivative and, optionally, a deaminase inhibitor. Products may be sprayed, powdered, sprinkled, misted, subjected to pressurizing conditions, submerged, coated or otherwise administered compositions of the invention to foster contact between the composition and the organism. Contact may be encouraged by incubating compositions of the invention with the product. For parasites, incubations may be performed at between about 0°C to about 50°C, preferably between about 4°C and about 37°C, and more preferably at about room temperature (18-22°C). For fungal and fungal-like organisms, incubations may be performed at between about 0°C to about 70°C, preferably between about 18°C and about 60°C, and more preferably at about 37°C or about 56°C. In either case, for example, the biological product, which may be living, is placed in a sterile container and sprayed or immersed in a solution or sprinkled with a powder containing an adenosine derivative and a deaminase inhibitor at an effective concentration. The deaminase inhibitor may not be necessary, for example, where there are no deaminases to inactivate. The product is maintained in this solution for a period of time necessary to effectively inactivate or destroy the organism. This time period may be minutes to weeks, but is preferably between about one minute to one week, more preferably between about one hour to one day. The product is than removed from the solution, washed if necessary, and utilized as desired. As compositions of the invention are generally safe and non-toxic, mmoval of product may not even be necessary, washing may not be necessary and the product may even be stored or shipped in the composition. In such cases, compositions of the invention may also contain additional components useful or desirable to accommodate the product during storage or shipping.

For example, biological products such as blood and blood products are required in vast quantities world-wide including areas of the world where parasitic and fungal diseases are endemic. Food and food products (salts and spices, sugar, molasses, sorghum, alimentary paste, dairy products, oils) including grains and vegetables (corn, wheat, rice, barley, peas, soybeans), breads, fruits (grapes, citrus fruits, bananas, apples, pears) and even fish and meats can be similarly treated. Maintaining an effective quantity of an adenosine derivative and, optionally, an effective quantity of a deaminase inhibitor in such supplies may prevent the spread of parasitic diseases from such products. Other products which are also required for medical uses include bone marrow and transplantable organs. Such products are typically obtained locally under emergency conditions. In such cases, there may be an undetected infection that would be passed to the patient receiving the product. Prophylactic treatment of these products would alleviate this risk and, as compositions of the invention are effective against a broad range of organisms, treatment only requires contact with this one composition. Multiple treatments would not be required or could be significantly reduced, increasing the overall chances of success for the therapy being administered to the patient.

Another embodiment of the invention comprises compositions for the treatment or prevention of a neoplastic disorder in a patient. The neoplastic disorder may be any disease or malady which could be characterized as a neoplasm, a tumor, a malignancy, a cancer or a disease which results in a relatively autonomous growth of cells. Composition contain an adenosine derivative such as a cordycepin or a cordycepin derivative, optionally, a deaminase inhibitor such as coformycin or deoxycoformycin, and a pharmaceutically acceptable carrier. The two agents may also be coupled together using techniques which are known to those skilled in the art.

In another embodiment of the invention, compositions of the invention may be used in any embodiment described herein in combination with other agents or therapies to maximize the effect of the compositions in an additive or synergistic manner. Patients are further administered another agent known to be effective against the neoplasia such as an alkylating agent, a purine or pyrimidine analog, a vinca or vinca-like alkaloid, an etoposide or etoposide-like drug, an antibiotic, a corticosteroid, a nitrosourea, an antimetabolite, a platinum based cytotoxic drug, a hormonal antagonist, an anti-androgen, an anti-estrogen, or a derivative, modification or combination of these agents. Therapies which are useful include augmention of conventional chemo-therapy, radiation therapy, antibody therapy, and other forms of therapy.

The following examples illustrate embodiments of the invention, but should not be viewed as limiting the scope of the invention.

### Example 1 Inhibitory Concentrations of 3'-Deoxynucleosides in Cell Cultures.

Cell cultures of various organisms were established in microtiter plates and maintained for a period of 24 hours after the addition of a 3'-deoxynucleoside (3'-dA, 3'-dG, 3'-dC, 3'-dU) (Calbiochem; San Diego, CA; Fluka Chemical; Ronkonkoma, NY; Pharmacia Biotech; Piscataway, NJ) or amphotericin B in µg/ml (Sigma Chemical; St. Louis, MO) as control. Results calculated as minimum inhibitory concentration (MIC) in micromoles per liter are shown in Table 6.

**Table 6**

| **MIC µM** | | | | | |
|---|---|---|---|---|---|
| Organism | 3'-dA | 3'-dC | 3'-dG | 3'-dU | Amphotericin B |
| *C. albicans* | 20 | >20 | >20 | >20 | <0.6 µg/ml |
| *C. tropicalis* | 20 | >20 | >20 | >20 | 1.25 µg/ml |
| *Rhizopus* | >20 | >20 | >20 | >20 | 2.50 µg/ml |
| *C. neoformans* | 20 | >20 | >20 | >20 | 2.50 µg/ml |
| *A. fumigatus* | 5 | 20 | >20 | >20 | 1.25 µg/ml |

From these results, deoxyadenosine (cordycepin) appears to demonstrate the most promise as an anti-fungal agent and inhibition observed is not non-specific nucleoside competition.

### Example 2 Mortality in Candida albicans Infected Mice.

Halen Sprague Dawley ICR mice were injected i.v. with approximately 10⁶ cfu of *Candida albicans*. Twenty-four hours after injection, mice were treated for 10 days with the indicated agents: 3'-deoxyadenosine (Sigma Chemical; St. Louis, MO) at 1.5 mg/kg/day i.p., deoxycoformycin (Sigma Chemical; St. Louis, MO) at 0.4 mg/kg/day i.p., amphotericin B (Sigma Chemical; St. Louis, MO) at 1.0 mg/kg/day i.p. Survival after 25 days is shown in Tables 7 and 8.

**Table 7**

| **Survival of Mice Treated with Nucleosides** | |
|---|---|
| Treatment | Survival After 25 Days |
| Control | 4/10 (40%) |
| 3'-deoxyadenosine | 3/10 (30%) |
| deoxycoformycin | 3/10 (30%) |
| 3'-deoxyadenosine + deoxycoformycin | 7/10(70%) |
| amphotericin B | 9/10 (90%) |

**Table 8**

| **Survival of Mice Treated with Nucleosides** | |
|---|---|
| Treatment | Survival After 15 Days |
| Control | 0/8 (00.0%) |
| 3'-Deoxyadenosine | 3/8 (37.5%) |
| Deoxycoformycin | 2/8 (25.0%) |
| 3'-Deoxyadenosine + Deoxycoformycin | 7/8 (87.5 %) |
| Amphotericin B | 4/8 (50.0%) |

The combination of an adenine derivative plus a deaminase inhibitor increased survival over 30% as compared to controls after 25 days of observation in the first experiment and greater than amphotericin B after 15 days of observation in the second experiment.

### Example 3 Time to Reach LD₅₀ of Various Agents in Mice.

Halen Sprague Dawley ICR mice were injected with approximately 10⁶ cfu *Candida albicans* and treated for 10 days with the following agents: 3'-deoxyadenosine (Sigma Chemical; St. Louis, MO) at 1.5 mg/kg/day i.p., deoxycoformycin (Sigma Chemical; St. Louis, MO) at 0.4 mg/kg/day i.p., amphotericin B (Sigma Chemical; St. Louis, MO) at 1.0 mg/kg/day i.p. Time in days to reach LD₅₀ is shown in Table 9.

**Table 9**

| **LD**_{**50**} **of Mice Treated with Nucleosides** | |
|---|---|
| Treatment | Days to Reach LD₅₀ |
| Control | 6 |
| 3'-Deoxyadenosine | 9.5 |
| Deoxycoformycin | 5 |
| 3'-Deoxyadenosine + Deoxycoformycin | > 30 |
| Amphotericin B | > 30 |

The LD₅₀ of combination treatments was comparable with treatment with amphotericin B which is known to be safe and effective.

Other embodiments and uses of the invention will be apparent to those skilled in the art from consideration of the specification and practice of the invention disclosed herein. It is intended that the specification and examples be considered exemplary only, with the true scope of the invention being indicated by the following claims.

## Claims

1. Use of an adenosine derivative comprising a 5-membered pentose ring coupled to a purine in the preparation of a pharmaceutical composition for the treatment of infection by fungal or fungal-like organisms in a patient.

2. Use of an adenosine derivative comprising a 5-membered pentose ring coupled to a purine in the preparation of a composition for the treatment or prevention of infection by fungal or fungal-like organisms in a biological product.

3. The use of claim 1 wherein the patient is a human.

4. The use of claim 1 wherein the patient is a mammal.

5. The use of claim 1 or 2 wherein the fungal organisms are selected from the group consisting of *Candida krusei, C. glabrata, C. albicans and C. tropicalis, C. parapsilosis, Trichophyton rubrum, T. mentagophytes, T. tonsurans*, *Microsporum audouini, M. canis and T. floccosum, Nocardia asteroides and N. brasiliensis, Actinomyces israelii,* species of the genera *Mucor, Absidia, Rhizopus, Cunninghamella* and other Zygomycetes, *Aspergillus fumigatus, A. flaws, A. niger* and other species of *Aspergillus, Cryptococcus neoformans*, *Paracoccidioides brasiliensis, Coccidioides immitis, Blastomycetes dermatitis* and *Histoplasma capsulatum.*

6. The use of claim 1 or 2 wherein the fungal-like organisms are a species of the genera *Actinomyces* or *Nocardia.*

7. The use of claim 2 wherein the biological product comprises whole blood, fractionated blood, plasma, serum, bone marrow or transplantable organs.

8. The use of claim 2 wherein the biological product is a food.

9. The use of claim 8 wherein the food product is selected from the group consisting of rice, wheat, barley, corn, soybeans, breads, oils, sugars, spices, dairy products, alimentary paste, vegetables and fruit.

10. The use of claim 2 wherein the biological product is derived from living cells.

11. The use of claim 10 wherein the biological product derived from living cells is selected from the group consisting of cytokines, antibodies, immune system regulators, recombinant proteins and blood products.

12. The use of claim I wherein the adenosine derivative is administered parenterally, sublingually, enterally, by pulmonary absorption or by topical application.

13. The use of claim 1 wherein a therapeutically effective amount of the adenosine derivative is between about 1 ng/kg patient weight to about 10 mg/kg patient weight.

14. The use of claim 1 or 2 wherein the adenosine derivative is a cordycepin or a cordycepin derivative.

15. The use of claim 1 or 2 wherein the adenosine derivative has the formula: 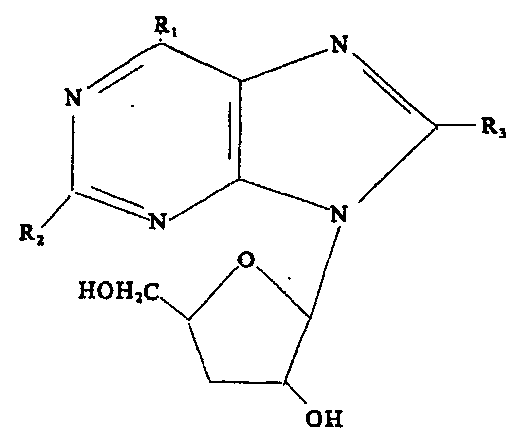 wherein R₁, R₂ and R₃ are each selected from the group consisting of hydrogen, hydroxyl, halides, alkyl and alkoxyl groups, amines and amides, sulfhydrals, nitryls, phosphoryls, sulfinyl and sulfonyl groups, and combinations and derivatives thereof, and R₁, R₂ and R₃ may be the same or different.

16. The use of claim 15 wherein R₁ is -OH, -SH, -SCH₃, -NHCH₃, R₂ is H, F, Cl, Br, I or NH₂; and R₃ is H, F, Cl, Br, I or CH₃.

17. The use of claim 1 or 2 further comprising the step of administering an effective amount of a deaminase inhibitor.

18. The use of claim 17 wherein the deaminase inhibitor is deoxycoformycin.

19. The use of claim 17 wherein the deaminase inhibitor is administered prior to the adenosine derivative.

20. The use of claim 17 wherein the deaminase inhibitor is administered together with said adenosine derivative.

21. The use of claim 17 wherein an effective amount of the deaminase inhibitor is between about 1 ng/kg patient weight to about 10 mg/kg patient weight.

22. The use of claim 17 wherein the adenosine derivative is administered in a two to twenty fold excess to the deaminase inhibitor.

23. The use of claim 17 wherein the adenosine derivative is coupled to the deaminase inhibitor.

24. The use of claim 17 further comprising administering an additional agent.

25. The use of claim 24 wherein the additional agent is selected from the group consisting of amphotericin B, sulfadiazine, flucytosine, mycoconazole, fluconazole, itraconazole, ketoconazole, griseofulvin, alkylating agents, purines and pyrimidine analogs, vinca and vinca-like alkaloids, etoposides and etoposide-like drugs, antibiotics such as penicillin, chlortetracycline, chloramphenicol and streptomycin, corticosteroids, nitrosoureas, antimetabolites, platinum based cytotoxic drugs, hormonal antagonists, anti-androgens, anti-estrogens and derivatives, modifications and combinations of these agents.

## Patentansprüche

1. Verwendung eines Adenosinderivats, das einen 5-gliedrigen Pentosering, der mit einem Purin verbunden ist, umfaßt, bei der Herstellung einer pharmazeutischen Zusammensetzung zur Behandlung einer Infektion durch Pilzorganismen oder pilzartige Organismen in einem Patienten

2. Verwendung eines Adenosinderivats, das einen 5-gliedrigen Pentosering, der mit einem Purin verbunden ist, umfaßt, bei der Herstellung einer Zusammensetzung zur Behandlung oder Vorbeugung einer Infektion durch Pilzorganismen oder pilzartige Organismen in einem biologischen Produkt.

3. Verwendung nach Anspruch 1, worin der Patient ein Mensch ist.

4. Verwendung nach Anspruch 1, worin der Patient ein Säugetier ist.

5. Verwendung nach einem der Ansprüche 1 oder 2, worin die Pilzorganismen ausgewählt sind aus der Gruppe, die aus Candida krusei, C. glabrata, C. albicans und C. tropicalis, C. parapsilosis, Trichophyton rubrum, T. mentagophytes, T. tonsurans, Microsporum audouini, M. canis und T. floccosum, Nocardia asteroides und N.brasiliensis, Actinomyces israelii, Spezies der Gattungen Mucor, Absidia, Rhizopus, Cunninghamella und andere Zygomyceten, Aspergillus fumigatus, A. flavus, A. niger und andere Spezies von Aspergillus, Cryptococcus neoformans, Paracoccidioides brasiliensis, Coccidioides immitis, Blastomycetes dermatitis und Histoplasma capsulatum besteht.

6. Verwendung nach einem der Ansprüche 1 oder 2, worin die pilzartigen Organismen eine Spezies der Gattungen Actinomyces oder Nocardia sind.

7. Verwendung nach einem der Ansprüche 1 oder 2, worin das biologische Produkt Vollblut, fraktioniertes Blut, Plasma, Serum, Knochenmark oder transplantierbare Organe umfaßt.

8. Verwendung nach Anspruch 2, worin das biologische Produkt ein Lebensmittel ist.

9. Verwendung nach Anspruch 8, worin das Lebensmittelprodukt ausgewählt ist aus der Gruppe, die aus Reis, Weizen, Gerste, Mais, Sojabohnen, Broten, Ölen, Zuckern, Gewürzen, Milchprodukten, Nahrungsergänzungspasten, Gemüsen und Früchten besteht.

10. Verwendung nach Anspruch 2, worin das biologische Produkt aus lebenden Zellen abstammt.

11. Verwendung nach Anspruch 10, worin das biologische Produkt, das aus lebenden Zellen abstammt, ausgewählt ist aus der Gruppe, die aus Cytokinen, Antikörpern, Immunsystemregulatoren, rekombinanten Proteinen und Blutprodukten besteht.

12. Verwendung nach Anspruch 1, worin das Adenosinderivat parenteral, sublingual, enteral, durch Absorption in der Lunge und durch lokales Auftragen verabreicht wird.

13. Verwendung nach Anspruch 1, worin die therapeutisch effektive Menge des Adenosinderivats zwischen etwa 1 ng/kg Patientengewicht und etwa 10 mg/kg Patientengewicht liegt.

14. Verwendung nach einem der Ansprüche 1 oder 2, worin das Adenosinderivat Cordycepin oder ein Cordycepinderivat ist.

15. Verwendung nach einem der Ansprüche 1 oder 2, worin das Adenosinderivat die Formel hat: 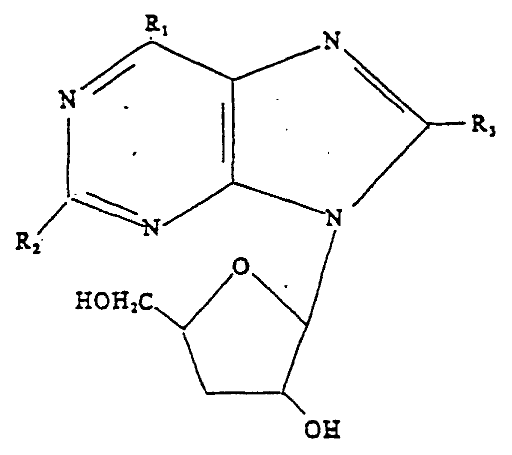 worin R1, R2 und R3, jeweils ausgewählt sind aus der Gruppe, die aus Wasserstoff, Hydroxyl, Halogeniden, Alkyl- und Alkoxygruppen, Aminen und Amiden, Hydrogensulfiden, Nitrylen, Phosphorylen, Sulfinyl- und Sulfonylgruppen und deren Kombinationen und Derivaten besteht, und R1, R2 und R3 gleich oder verschieden sein können.

16. Verwendung nach Anspruch 15, worin R1 -OH, -SH, -SCH3, -NHCH3 ist, R2 H, F, Cl, Br, I oder NH2 ist, und R3 H, F, Cl, Br, I oder CH3 ist.

17. Verwendung nach einem der Ansprüche 1 oder 2, die weiterhin den Schritt der Verabreichung einer effektiven Menge eines Deaminase-Inhibitors umfaßt.

18. Verwendung nach Anspruch 17, worin der Inhibitor Deoxycoformycin ist.

19. Verwendung nach Anspruch 17, worin der Deaminase-Inhibitor vor dem Adenosinderivat verabreicht wird.

20. Verwendung nach Anspruch 17, worin der Deaminase-Inhibitor zusammen mit dem Adenosinderivat verabreicht wird.

21. Verwendung nach Anspruch 17, worin eine effektive Menge des Deaminase-Inhibitors zwischen etwa 1 ng/kg Patientengewicht und etwa 10 mg/kg Patientengewicht liegt.

22. Verwendung nach Anspruch 17, worin das Adenosinderivat in einem zweibis zwanzigfachen Überschuß des Deaminase-Inhibitors verabreicht wird.

23. Verwendung nach Anspruch 17, worin das Adenosinderivat an den Deaminase-Inhibitor gebunden ist.

24. Verwendung nach Anspruch 17, die weiterhin die Verabreichung eines zusätzlichen Mittels umfaßt.

25. Verwendung nach Anspruch 24, worin das zusätzliche Mittel ausgewählt ist aus der Gruppe, die aus Amphotericin B, Sulfadiazin, Flucytosin, Mycoconazol, Fluconazol, Itraconazol, Ketoconazol, Griseofulvin, Alkylierungsmitteln, Purinen und Pyrimidinanalogen, Vincaalkaloiden und vincaartigen Alkaloiden, Etoposidmedikamenten und etoposidartigen Medikamenten, Antibiotika, wie beispielsweise Penicillin, Chlortracyclin, Chloramphenicol und Streptomycin, Corticosteroiden, Nitrosoharnstoffen, Antimetaboliten, platinbasierten cytotoxischen Medikamenten, hormonellen Antagonisten, Antiandrogenen, Antiöstrogenen und Derivaten sowie Modifikationen und Kombinationen dieser Mittel besteht.

## Revendications

1. Utilisation d'un dérivé de l'adénosine comprenant un cycle pentose pentagonal attaché à une purine pour la préparation d'une composition pharmaceutique pour le traitement de l'infection d'un patient par des organismes fongiques ou analogues.

2. Utilisation d'un dérivé de l'adénosine comprenant un cycle pentose pentagonal attaché à une purine pour la préparation d'une composition pour le traitement ou la prévention de l'infection d'un produit biologique par des organismes fongiques ou analogues.

3. Utilisation de la revendication 1,
**caractérisée en ce que**
le patient est un humain.

4. Utilisation de la revendication 1,
**caractérisée en ce que**
le patient est un mammifère.

5. Utilisation de la revendication 1 ou 2,
**caractérisée en ce que**
les organismes fongiques sont choisis dans le groupe comprenant *Candida krusei, C.glabrata, C.albicans et C.tropicalis, C. parapsilorin, Trichophyton rubrum, T. mentagophytes, T. tonsurans, Microsporum audouini, M.canis et T.floccosum, Nocardia asteroides et Nbrosiliensis, Actinomyces israellii,* des espèces du genre *Mucor, Absidia, Rhizopus, Cunninghamella* et autres Zygomycétes, *Aspergillus fumigatus, AJlavus, A.niger* et autres espèces de *Aspergillus, Cryptococcus neoformans, Paracoccidioides brasiliensis, Coccidioides immitis, Blastomycetes dermatitis* et *Histoplasma capsulatum.*

6. Utilisation de la revendication 1 ou 2,
**caractérisée en ce que**
les organismes identiques aux organismes fongiques sont des espèces du genre *Actinomyces* ou *Nocardia.*

7. Utilisation de la revendication 2,
**caractérisée en ce que**
le produit biologique comprend du sang complet, du sang fractionné, du plasma, du sérum, de la moelle osseuse ou des organes transplantables.

8. Utilisation de la revendication 2,
**caractérisée en ce que**
le produit biologique est un produit comestible.

9. Utilisation de la revendication 8,
**caractérisée en ce que**
le produit comestible est choisi dans le groupe comprenant le riz, le blé, l'orge, le maîs, les graines de colza, des pains, des huiles, des sucres, des épices, des produits laitiers, des pâtes alimentaires, des légumes et des fruits.

10. Utilisation de la revendication 2,
**caractérisée en ce que**
le produit biologique provient de cellules vivantes.

11. Utilisation de la revendication 10,
**caractérisée en ce que**
le produit biologique provenant de cellules vivantes est choisi dans le groupe comprenant des cytokines, des anticorps, des régulateurs du système immunitaire, des protéines recombinantes et des produits sanguins.

12. Utilisation de la revendication 1,
**caractérisée en ce que**
le dérivé de l'adénosine est administré parentérallement, sous la langue, entérallement, par absorption pulmonaire ou par application locale.

13. Utilisation de la revendication 1,
**caractérisée en ce que**
la quantité thérapeutiquement efficace du dérivé de l'adénosine est comprise entre environ 1 ng/kg du poids du patient et environ 10 mg/kg du poids du patient.

14. Utilisation de la revendication 1 ou 2,
**caractérisée en ce que**
le dérivé de l'adénosine est une cordycèpine ou un dérivé de cordycèpine.

15. Utilisation de la revendication 1 ou 2,
**caractérisée en ce que**
le dérivé de l'adénosine a la formule ; 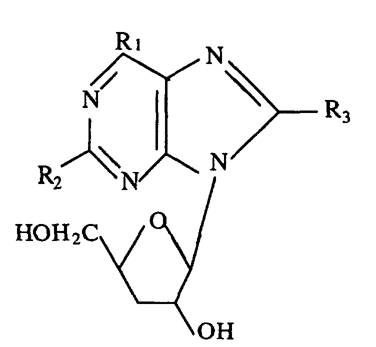 **caractérisée en ce que**
R₁, R₂ et R₃ sont choisis, chacun, dans le groupe comprenant un hydrogène, un hydroxyle, des halogènures, des groupes alkyl et alkoxy, des amines et des amides, des sulfhyrdates, des nitriles, des phosphoryles, des groupes sulfinyle et sulfonyle et des combinaisons et des dérivés de ceux-ci ; R₁, R₂ et R₃ pouvant être identiques ou différents.

16. Utilisation de la revendication 15,
**caractérisée en ce que**
R₁ est -OH, -SH, -SCH₃, -NHCH₃; R₂ est H, F, Cl. Br, I ou NH₂ ; et R₃ est H, F, Cl, Br, I ou CH₃.

17. Utilisation de la revendication 1 ou 2, comprenant en plus l'étape d'administration d'une quantité efficace d'un inhibiteur de désaminase.

18. Utilisation de la revendication 17,
**caractérisée en ce que**
l'inhibiteur de désaminase est la désoxycoformycine.

19. Utilisation de la revendication 17,
**caractérisée en ce que**
l'inhibiteur de désaminase est administré avant le dérivé de l'adénosine.

20. Utilisation de la revendication 17,
**caractérisée en ce que**
l'inhibiteur de désaminase est administré en même temps que le dérivé de l'adénosine.

21. Utilisation de la revendication 17,
**caractérisée en ce que**
la quantité efficace d'inhibiteur de désaminase est comprise entre environ 1 ng/kg du poids du patient et environ 10 mg/kg du poids du patient.

22. Utilisation de la revendication 17,
**caractérisée en ce que**
le dérivé de l'adénosine est administré en excès de deux à vingt fois par rapport à l'inhibiteur de désaminase.

23. Utilisation de la revendication 17,
**caractérisée en ce que**
le dérivé de l'adénosine est attaché à l'inhibiteur de désaminase.

24. Utilisation de la revendication 17 comprenant en plus l'administration d'un agent supplémentaire.

25. Utilisation de la revendication 24,
**caractérisée en ce que**
l'agent supplémentaire est choisi dans le groupe comprenant amphotéricine B, sulfadiazine, flucytosine, mycoconazole, fluconazole, itraconazole, kétoconazole, griséofulvine, agents d'alkylation, les purines et pyrimidines analogues, des alcaloïdes vinca et analogues, médicaments étoposides et analogues, des antibiotiques comme pénicilline, chlorotétracycline, chloramphénicol et streptomycine, corticostéroïdes, nitrosurées, antimétabolites, remèdes cytoxiques à base de platine, des anatagonistes hormonaux des anti-androgènes, anti-oestrogènes et dérivés, formes modifiées ou combinaisons de ces agents.
